# EUROPEAN PATENT APPLICATION

(11) **EP 4 629 254 A1**
(43) Date of publication of application: **08.10.2025**
(21) Application number: 25163068.7
(22) Date of filing: 11.03.2025
(51) Int. Cl.: G16H 10/60, G16H 20/00, G16H 20/40, G16H 40/63, A61B 6/00, A61B 6/46

(54) **SYSTEMS AND METHODS FOR RADIATION DOSE MANAGEMENT**

(30) Priority: 05.04.2024 US 202418628539
(71) Applicant: GE Precision Healthcare LLC, Waukesha, WI 53188 (US)
(72) Inventor: GALLO, Alexandre, 67000 Strasbourg (FR); PUJADAS, María del Pilar, 28023 Madrid (ES); NOLAN, Jason, Chicago, 60661 (US); METTER ROTHAN, Jeremie, 67000 Strasbourg (FR)
(74) Representative: Kilburn & Strode LLP

(57) **Abstract**

Systems and methods are herein provided for radiation dose management. In one example, a computing device comprising a display screen displays on the display screen a menu listing one or more data repositories of one or more patients, and additionally displays on the display screen a radiation timeline within a graphical user interface (GUI) accessible from the menu, wherein the radiation timeline displays, patient data including one or more examination phases and radiation data thereof of a selected imaging examination, the radiation data and one or more examination phases obtained from the one or more data repositories, wherein each examination phase is plotted in chronological order and a component is plotted according to a timestamp and a scan range of a corresponding examination phase, and wherein the GUI is displayed while the one or more data repositories are in an un-launched state.

## Description

### FIELD

Embodiments of the subject matter disclosed herein relate to radiation dose management, and more particularly to a graphical timeline for radiation dose management.

### BACKGROUND

Imaging examinations for patients often include multiple steps or phases, including administration of injected agents, multiple series of image acquisitions, and the like. For some imaging types, one or more phases may include delivery of radiation, either for image acquisition or during administration of injected agents. Radiation, while necessary for image acquisition in certain circumstances, needs to be closely monitored by care providers and technicians to ensure proper dose administration and compliance with recommended care guidelines. Many hospital systems and clinicians operate by the As Low As Reasonably Achievable (ALARA) principle in order to minimize radiation exposure to patients while still obtaining the necessary diagnostic information. Radiation dose management software aims to record and present radiation dose data to aid clinicians in monitoring and analyzing radiation dose data of patients.

### BRIEF DESCRIPTION

In one example, a computing device comprising a display screen, the computing device being configured to display on the display screen a menu listing one or more data repositories of one or more patients, and additionally being configured to display on the display screen a radiation timeline within a graphical user interface (GUI) accessible from the menu, wherein the radiation timeline displays, for each patient, patient data including one or more examination phases and radiation data thereof of a selected imaging examination, the radiation data and one or more examination phases obtained from the one or more data repositories, wherein each examination phase is plotted along a horizontal axis in chronological order and a component is plotted within the radiation timeline according to a timestamp of a corresponding examination phase and a scan range of the corresponding examination phase, and wherein the GUI is displayed while the one or more data repositories are in an un-launched state.

It should be understood that the brief description above is provided to introduce in simplified form a selection of concepts that are further described in the detailed description. It is not meant to identify key or essential features of the claimed subject matter, the scope of which is defined uniquely by the claims that follow the detailed description. Furthermore, the claimed subject matter is not limited to implementations that solve any disadvantages noted above or in any part of this disclosure.

### BRIEF DESCRIPTION OF THE DRAWINGS

The present invention will be better understood from reading the following description of non-limiting embodiments, with reference to the attached drawings, wherein below:
FIG. 1 shows a block diagram of an example system for generating and displaying a radiation dose timeline in a timeline graphical user interface (GUI);
FIG. 2 shows a portion of an example radiation timeline;
FIG. 3A shows a portion of another example radiation timeline;
FIG. 3B shows a portion of another example radiation timeline;
FIG. 4 shows an example of a secondary radiation timeline;
FIG. 5 shows an example radiation timeline;
FIG. 6 shows another example of a radiation timeline including a blind acquisition element;
FIG. 7 shows an example radiation timeline with isotope activity elements;
FIG. 8 shows an example component of a radiation timeline;
FIG. 9 shows another example radiation timeline;
FIG. 10 shows a first example GUI displaying a radiation timeline;
FIG. 11 shows a second example GUI displaying a radiation timeline; and
FIG. 12 shows a flowchart illustrating a method for generating a radiation timeline.

### DETAILED DESCRIPTION

The following description relates to various embodiments of radiation dose management. In particular, systems and methods for generating one or more radiation timelines for a selected imaging examination, the one or more radiation timelines being displayed in a graphical user interface (GUI), are provided.

In medical imaging, many modalities employ radiation for imaging internal organs of subjects (e.g., patients), such as computed tomography (CT) imaging and positron emission tomography (PET) imaging, for diagnostic purposes, disease monitoring purposes, and the like. Medical professionals operate by the As Low As Reasonably Achievable (ALARA) principle in order to minimize radiation exposure to patients while still obtaining the necessary diagnostic information. Thus, the amounts of irradiation delivered to patients for individual imaging examinations and cumulatively over time are monitored by medical professionals both to minimize radiation exposure as well as to ensure proper delivery by imaging systems.

Radiation dose management software aims to record and monitor the amount of irradiation received by patients during imaging examinations such as CT, mammography, radio fluoroscopy (RF), PET, x-ray, etc. To do so, data are received from the imaging systems and reported by the software. As many types of medical imaging exist, including those that use irradiation, the data are heterogeneous and examinations are considered depending on modality type, thereby limiting ability to cumulatively consider radiation information. Further, current radiation dose management software reports radiation data in a tabular manner but does not provide insights to users, thereby reducing the practical usage of such systems. Tabular data displays detailed information of examinations but may not help to visualize easily the actual flow of the examination phase by phase. This is especially the case when an imaging examination combines modalities, such as PET-CT imaging, in which, in traditional radiation dose management software data of the modalities therein is reported separately.

Systems and methods for radiation dose management that addresses at least some of these issues with traditional radiation dose management software, including generating and presenting data of imaging examinations in graphical radiation timelines in a GUI, is herein presented. As will be described herein, for a selected imaging examination, data is received from one or more data repositories, including relevant imaging systems, and one or more radiation timelines are displaying within the GUI. The radiation timelines plot phases of the selected imaging examination in a chronological manner. Components corresponding to one or more of the examination phases are plotted within the timeline according to the scan range (e.g., range of z-coordinates of the patient imaged) of each examination phase. In this way, the graphical timelines allow for visualization of radiation dose data, including areas of irradiation and amounts of irradiation to different areas of the patient's body.

Further, alert elements may be displayed within the timeline based on the data from the one or more data repositories that indicate to the user potential issues, including over-irradiation, practice issues, image quality estimations, and the like. Further still, data of radioactivity of injected isotopes, for relevant imaging modalities, may also be displayed within the radiation timeline. Thus, a unified user interface display is presented that presents all relevant information in a visual manner to increase ease of analysis for the user.

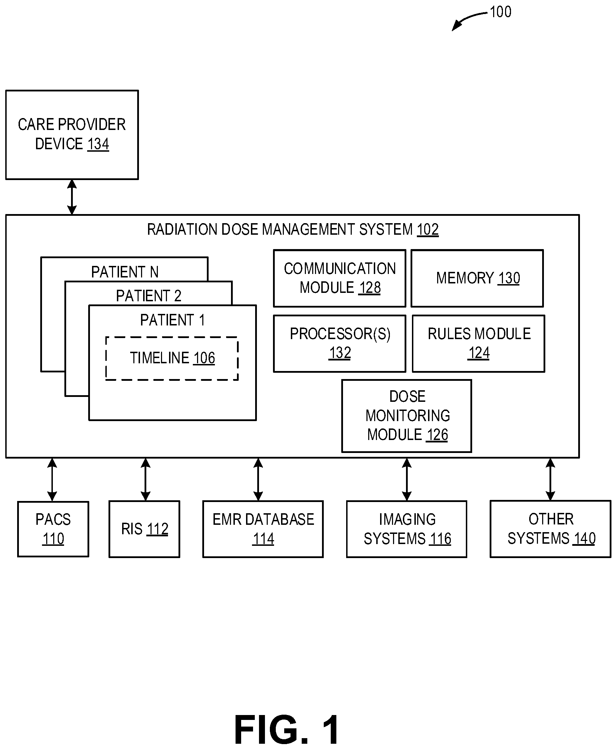

Embodiments of the present disclosure will now be described, by way of example, with reference to the figures. Starting with FIG. 1, an example of a patient information system 100 that may be implemented in a medical facility such as a hospital is shown. Patient information system 100 may be a portion of or otherwise included in a computing device and/or computing system. Patient information system 100 may include a radiation dose management system 102. The radiation dose management system 102 may include resources (e.g., memory 130, processor(s) 132) that may be allocated to store and execute timelines for each of a plurality of patients. For example, as shown in FIG. 1, timeline 106 is stored on radiation dose management system 102 for a first patient (patient 1); a plurality of additional timelines may be stored on and/or generated by radiation dose management system 102, each corresponding to a respective patient (patient 2 up to patient N) and in some examples more than one timeline may correspond to one patient as each timeline is specific to a given imaging examination.

Each timeline 106 may include graphical representations of imaging examination phases arranged chronologically, as will be described further below. The imaging examination phases depicted on the timeline 106 may include image series acquisitions, scout image acquisitions, injected agent administrations, monitoring phases, radiation dose amounts for respective acquisitions, isotope activity of injected agents, and the like. Further, the patient medical information, including iodine injection, saline injection, radiation dose amounts, and isotope activity, may be analyzed by dose monitoring module 126, which may be used to generate and output alerts to the timeline GUI when an irradiation exceeds a preset threshold.

The patient information that is presented via the timeline 106 may be stored in different medical databases or storage systems (e.g., data repositories) in communication with radiation dose management system 102. For example, as shown, the radiation dose management system 102 may be in communication with a picture archiving and communication system (PACS) 110, a radiology information system (RIS) 112, an electronic medical record (EMR) database 114, and one or more imaging systems 116. PACS 110 may store medical images and associated information thereof, including imaging reports (e.g., clinician findings), series data, and the like. PACS 110 may store images and communicate according to digital imaging and communications in medicine (DICOM) format. RIS 112 may store radiology images and associated reports, such as CT images, x-ray images, MRI images, etc. EMR database 114 may store electronic medical records for a plurality of patients. EMR database 114 may be a database stored in a mass storage device configured to communicate with secure channels (e.g., HTTPS and TLS), and store data in encrypted form. Further, the EMR database may be configured to control access to patient electronic medical records such that only authorized healthcare providers may edit and access the records. An EMR for a patient may include patient demographic information, family medical history, past medical history, lifestyle information, preexisting medical conditions, current medications, allergies, surgical history, past medical screenings and procedures, reports from past hospitalizations, prior imaging reports, and the like. The imaging systems 116 may comprise CT imaging systems, MRI systems, x-ray systems, nuclear medicine systems such as single photon emission computerized tomography (SPECT) and PET systems, RF systems, others, and/or combinations thereof. In some examples, one or more of the imaging systems 116 may comprise subsystems therein that control, determine, or otherwise monitor and record the amount of radiation or contrast administered per each examination session and in each series acquired during the examination session. For example, a CT system may include a control system with embedded software that determines settings of the system, such as tube current and tube voltage that may affect the amount of radiation delivered during the scan. Further, the CT system may include an automatic exposure control system that adjust such settings according to the scan protocol and patient body size. In some examples, an imaging system may comprise dose modulation software configured to adjust radiation exposure during a scan. The imaging systems 116 may thus monitor and record an amount of radiation delivered during a scan. The imaging systems 116 may thereby store in memory radiation data for one or more examinations and phases thereof.

Further, the radiation dose management system 102 may be in communication with one or more other systems 140. The one or more other systems 140 may include iodine injectors, nuclear medicine administration information systems, and the like, that may store and/or record information of injected agents, including radioactive agents, iodine contrast agents, saline, and the like.

When requested, timeline 106 may be displayed on the one or more display devices. As shown in FIG. 1, a care provider device 134, and in some examples more than one care provider device, may be communicatively coupled to radiation dose management system 102. Each care provider device may include a processor, memory, communication module, user input device, display (e.g., screen or monitor, and/or other subsystems and may be in the form of a desktop computing device, a laptop computing device, a tablet, a smart phone, or other device). Each care provider device may be adapted to send and receive encrypted data and display medical information, including medical images in a suitable format such as DICOM or other standards. The care provider devices may be located locally at the medical facility (such as in a room of a patient or a clinician's office) and/or remotely from the medical facility (such as a care provider's mobile device). The timeline 106 may be displayed within a GUI when the one or more data repositories are in an un-launched state, meaning that the data repositories are not current accessed by the display device or care provider device.

When viewing timeline 106 via a display of a care provider device, a care provider may enter input (e.g., via the user input device, which may include a keyboard, mouse, microphone, touch screen, stylus, or other device) that may be processed by the care provider device and sent to the radiation dose management system 102. In examples where the user input is a selection of a link or user interface control button/element of the timeline, the user input may trigger display of a selected EMR, trigger display of a timeline for a specified imaging examination, trigger display of a desired point in time or view of the displayed timeline (e.g., scroll to a later point in time, trigger display of various types of information within the timeline (e.g., such as blind acquisition information, as will be further described below), trigger display of additional information regarding a particular examination phase, or other actions.

The radiation dose management system 102 includes the dose monitoring module 126 that may be configured to analyze patient data obtained from the plurality of sources (e.g., PACS 110 and the imaging systems 116). The dose monitoring module 126 may be configured to analyze patient data relevant to radiation delivery, such as examinations, examination phases thereof, and radiation amounts for each examination phases. In examples in which the imaging examination is a nuclear medicine examination that includes administration of a radioactive isotope, such as 18-Fluorine, the patient data may also include information of radioactivity of the radioactive isotope during the examination and at relevant phases. In some examples, the initial radioactivity level of the radioactive isotope upon administration may be obtained from an external system and based thereon, the dose monitoring module 126 may determine amount of radioactivity at subsequent timestamps, for example based on known radioactive decay parameters.

The dose monitoring module 126 may be in communication with a rules module 124. The rules module 124 may include rule sets and criteria for one or more dosage and examination scenarios, for example rules for expected radiation dose amounts for a given examination, recommended radiation dose amounts for a patient over a period of time, threshold radiation dose amounts, etc. The criteria may include one or more triggers that when satisfied, cause the dose monitoring module 126 to generate an alert. For example, when the patient data meets criteria for exceeding a threshold radiation dose amount for a given examination phase, set of phases, or examination session as defined by the rules module 124, the dose monitoring module 126 may generate an alert. In some examples, the alert may be displayed within the timeline at a respective timestamp, as will be further described below.

Radiation dose management system 102 may further include a communication module 128, memory 130, and processor(s) 132 to store and generate timelines, as well as send and receive communications, GUIs, medical/imaging data, and other information.

Communication module 128 facilitates transmission of electronic data within and/or among one or more systems. Communication via communication module 128 may be implemented using one or more protocols. In some examples, communication via communication module 128 occurs according to one or more standards (e.g., DICOM, Health Level Seven (HL7), ANSI X12N, etc.). Communication module 128 can be a wired interface (e.g., a data bus, a Universal Serial Bus (USB) connection, etc.) and/or a wireless interface (e.g., radio frequency, infrared, near field communication (NFC), etc.). For example, communication module 128 may communicate via a wired local area network (LAN), wireless LAN, wide area network (WAN), etc. using any past, present, or future communication protocol (e.g., BLUETOOTH^{™}, USB 2.0, USB 3.0, etc.).

Memory 130 may include one or more data storage structures, such as optical memory devices, magnetic memory devices, or solid-state memory devices, for storing programs and routines executed by processor(s) 132 to carry out various functionalities disclosed herein. Memory 130 may include any desired type of volatile and/or non-volatile memory such as, for example, static random access memory (SRAM), dynamic random access memory (DRAM), flash memory, read-only memory (ROM), etc. Processor(s) 132 may be a multi-processor system, and thus, may include one or more additional processors that are identical or similar to each other and that are communicatively coupled via an interconnection bus. As an example, the dose monitoring module 126 may store instructions for generating alerts in the memory 130 that are executable by the processor(s) 132.

As used herein, the terms "sensor," "system," "unit," or "module" may include a hardware and/or software system that operates to perform one or more functions. For example, a sensor, module, unit, or system may include a computer processor, controller, or other logic-based device that performs operations based on instructions stored on a tangible and non-transitory computer readable storage medium, such as a computer memory. Alternatively, a sensor, module, unit, or system may include a hard-wired device that performs operations based on hard-wired logic of the device. Various modules or units shown in the attached figures may represent the hardware that operates based on software or hardwired instructions, the software that directs hardware to perform the operations, or a combination thereof.

"Systems," "units," "sensors," or "modules" may include or represent hardware and associated instructions (e.g., software stored on a tangible and non-transitory computer readable storage medium, such as a computer hard drive, ROM, RAM, or the like) that perform one or more operations described herein. The hardware may include electronic circuits that include and/or are connected to one or more logic-based devices, such as microprocessors, processors, controllers, or the like. These devices may be off-the-shelf devices that are appropriately programmed or instructed to perform operations described herein from the instructions described above. Additionally, or alternatively, one or more of these devices may be hard-wired with logic circuits to perform these operations.

Thus, radiation dose management system 102 may be configured to obtain/ingest medical and medical imaging data from a variety of sources (e.g., PACS, RIS, imaging systems, etc.) and analyze, extract, and register selected data to generate a timeline and elements such as alerts for each patient as described herein. In some examples, radiation dose management system 102 may include one or more data filters configured to monitor and filter the ingested data to ensure that only relevant and complete data is presented in the timeline. In some examples, certain types of data may be presented in one timeline (e.g., time-stamped data) and other types of data (e.g., data entries without timestamps) may be presented in a secondary timeline within the same GUI.

One or more of the devices described herein may be implemented over a cloud or other computer network. For example, radiation dose management system 102 is shown in FIG. 1 as constituting a single entity, but it is to be understood that radiation dose management system 102 may be distributed across multiple devices, such as across multiple servers. Further, while the elements of FIG. 1 are shown as being housed at a single medical facility, it is to be appreciated that any of the components described herein (e.g., EMR database, RIS, PACS, etc.) may be located off-site or remote from the radiation dose management system 102. Further, the longitudinal data utilized by the radiation dose management system 102 for the timeline generation and other tasks described below could come from systems within the medical facility or obtained through electronic means (e.g., over a network) from other referring institutions.

While not specifically shown in FIG. 1, additional devices described herein (e.g., care provider device 134) may likewise include user input devices, memory, processors, and communication modules/interfaces similar to communication module 128, memory 130, and processor(s) 132 described above, and thus description of communication module 128, memory 130, and processor(s) 132 likewise applies to the other devices described herein. As an example, the care provider devices (e.g., care provider device 134) may store user interface templates in memory that include placeholders for relevant information stored on radiation dose management system 102 or sent via radiation dose management system 102. For example, care provider device 134 may store a user interface template for a radiation timeline that a user of a care provider device 134 may configure with placeholders for desired patient information. When the timeline is displayed on the care provider device, the relevant patient information may be retrieved from radiation dose management system 102 and inserted in the placeholders. The user input devices may include keyboards, mice, touch screens, microphones, and other suitable devices.

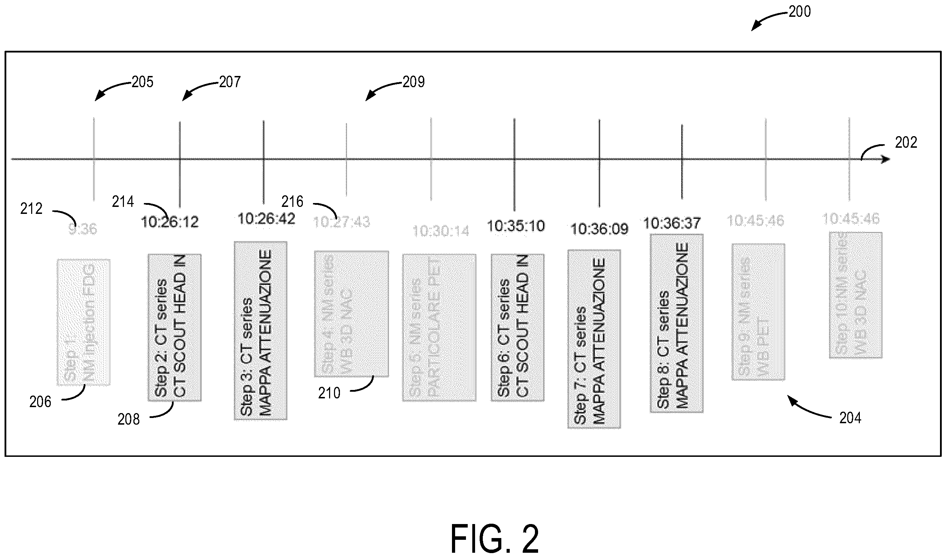

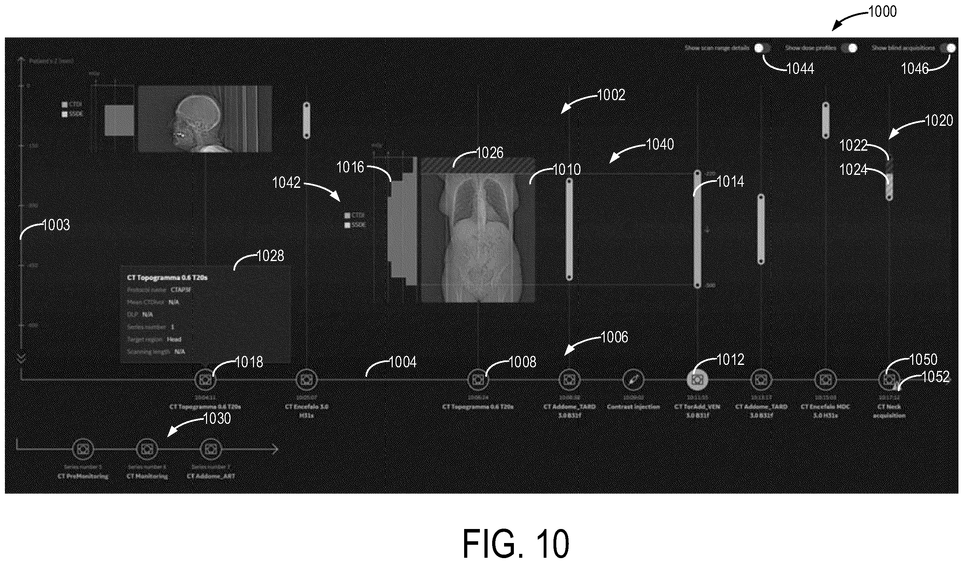

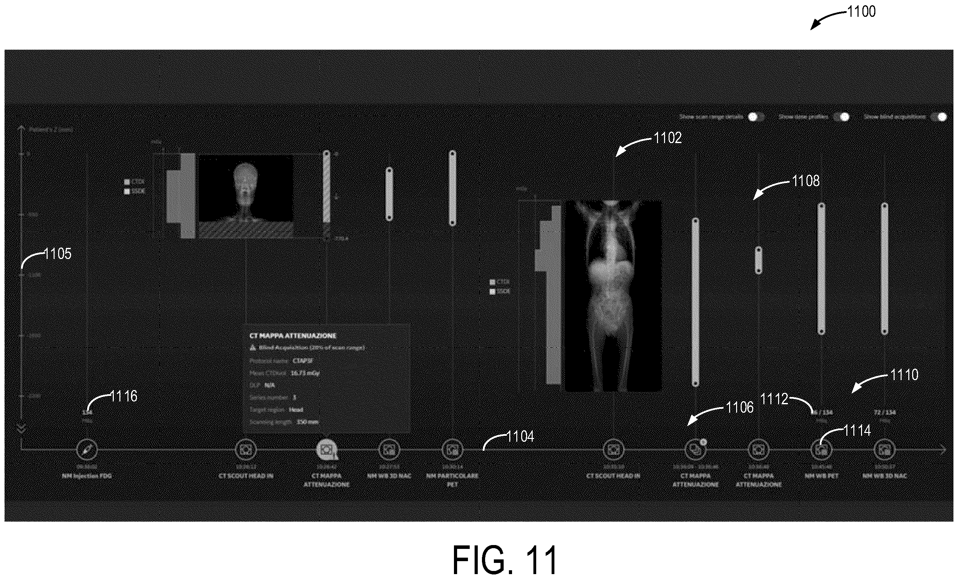

Turning now to FIG. 2, an example of a portion of a radiation timeline 200 is shown that may be generated for a patient for a given imaging examination by radiation dose management system 102. The radiation timeline 200 may be displayed within a GUI of a radiation dose management system (e.g., radiation dose management system 102), which may be displayed on a display of a care provider device (e.g., care provider device 134 of FIG. 1). Radiation timeline 200 may include a plurality of components, as will be further described below, that may be displayed individually or at the same time. Components and icons displayed within and/or on the radiation timeline 200 may indicate or represent various phases of the selected imaging examination and radiation dose data thereof retrieved from a plurality of data repositories (e.g., PACS, imaging systems, etc.). The radiation timeline 200 as shown in FIG. 2 may be displayed within a GUI, as will be described further with respect to FIGS. 10 and 11.

The portion of the radiation timeline 200 depicted in FIG. 2 includes a horizontal axis 202. The horizontal axis 202 may indicate time from left to right and may comprise a plurality of examination phases 204 plotted thereon. Each of the plotted examination phases 204 may be plotted as icons, in some examples also comprising an examination phase label and a timestamp. For example, a first examination phase icon 205 may comprise a first examination phase label 206 and a first timestamp 212, a second examination phase icon 207 may comprise a second examination phase label 208 and a second timestamp 214, and a third examination phase icon 209 may comprise a third examination phase label 210 and a third timestamp 216. In some examples, the timestamps displayed along the horizontal axis 202 that correspond to image acquisitions may indicate a timestamp of the first image in the dataset of the corresponding acquisition.

The plotted examination phases of the horizontal axis 202 may be arranged chronologically, with phases occurring earlier being arranged towards a left hand side of the horizontal axis 202 and phases occurring later being arranged towards a right hand side. For example, the first examination phase represented by the first examination phase icon 205 may have occurred before the second and third examination phases represented by the second and third examination phase icons 207, 209 as the first examination phase icon 205 is plotted further to the left on the horizontal axis 202 than the second and third examination phase icons 207, 209. Further, the first timestamp 212 may correspond to an earlier time than the second and third timestamps 214, 216. The horizontal axis 202 may not plot examination phases in a linear, chronologically proportional manner, in some examples. Rather, the examination phases may be plotted along the horizontal axis 202 so as to fit a window of the radiation timeline 200 when it is displayed within a GUI, as will be further described below.

Additionally, each of the examination phases may correspond to phases of the selected imaging examination. Thus, for imaging examinations that include multiple modalities, for example a PET-CT scan, phases may correspond to different modality phases. For example, the first examination phase 205 may correspond to a nuclear medicine injection of 18-Fluorine while the second examination phase may correspond to a CT scout acquisition. In some examples, phases corresponding to different modalities may be displayed within the GUI with different colors or different icon types.

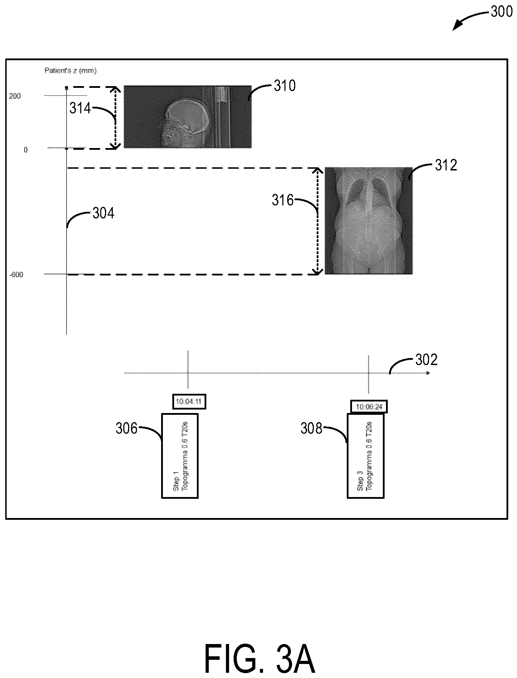

Turning now to FIG. 3A, a first example radiation timeline 300 including both a horizontal axis and a vertical axis is shown. As described with respect to FIG. 2, the radiation timeline 300 may be displayed within a GUI in response to user input, for example user selection of an imaging examination. The radiation timeline 300 may be displayed within a GUI of a radiation dose management system (e.g., radiation dose management system 102), which may be displayed on a display of a care provider device (e.g., care provider device 134 of FIG. 1). Radiation timeline 300 may include a plurality of components, as will be further described below, that may be displayed individually or at the same time. Components and icons displayed within and/or on the radiation timeline 300 may indicate or represent various phases of the selected imaging examination and radiation dose data thereof retrieved from a plurality of data repositories (e.g., PACS, imaging systems, etc.).

The radiation timeline 300 may comprise a horizontal axis 302 and a vertical axis 304. The horizontal axis 302, similar to as described with respect to FIG. 2, may have a plurality of examination phases plotted thereon. The vertical axis 304 may indicate z-coordinates of the patient, for example in mm. The radiation timeline 300 may display various components, plotted according to time aligned with a corresponding examination phase of the horizontal axis 302, and scan range (e.g., range of z-coordinates scanned of the patient) in which they were obtained along the vertical axis 304. In some examples, the components displayed within the radiation timeline 300 may be representations of scout image acquisitions that may be used as a reference for an expected scan range for subsequent examination phases.

For example, the horizontal axis 302 comprises a first examination phase icon 306 and a second examination phase icon 308. The first examination phase icon 306 may comprise a first examination phase label and a first timestamp and the second examination phase icon 308 may comprise a second examination phase label and a second time stamp. The first examination phase icon 306 may correspond to a first scout acquisition and the second examination phase icon 308 may correspond to a second scout acquisition. The first scout acquisition may be represented within the radiation timeline 300 by a first scout image component 310 and the second scout acquisition may be represented by a second scout image component 312.

In the example shown in FIG. 3A, the first scout acquisition may be a scan of a head while the second scout acquisition may be a scan of a torso. The first scout acquisition may have a first scan range 314 and the second scout acquisition may have a second scan range 316. The first and second scan ranges 314, 316 may correspond to ranges of z-coordinates, as plotted on the vertical axis 304. Thus, the various scout components may be plotted within the radiation timeline 300 in a relative manner, whereby the position along the vertical axis corresponds to the scan range of given examination phases. Further, as the two scout images may represent two areas of the body, the relationship of the scouts to each other as plotted along the vertical axis may correlate to the anatomical positions of the regions imaged by each scout acquisition.

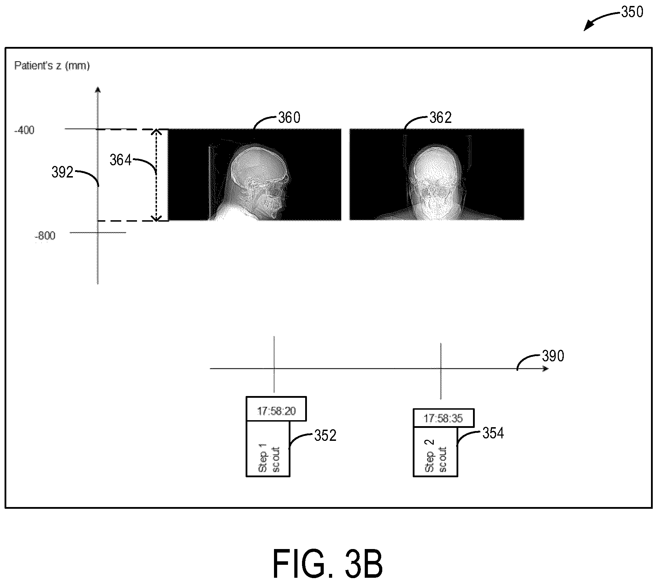

As another example, FIG. 3B shows another example radiation timeline 350 including both a horizontal axis and a vertical axis. As described with respect to FIGS. 2 and 3A, the radiation timeline 350 may be displayed within a GUI in response to user input, for example user selection of an imaging examination listed in a previous GUI. The radiation timeline 350 may be displayed within a GUI, which may be displayed on a display of a care provider device (e.g., care provider device 134 of FIG. 1). Components and icons displayed within and/or on the radiation timeline 350 may indicate or represent various phases of the selected imaging examination and radiation dose data thereof retrieved from a plurality of data repositories (e.g., PACS, imaging systems, etc.).

Similar to as described for the radiation timeline 300, the radiation timeline 350 comprises a horizontal axis 390 and a vertical axis 392. One or more examination phases may be plotted on the horizontal axis 390. For example, a first examination phase icon 352 and a second examination phase icon 354 may be plotted along the horizontal axis 390. The first examination phase may be a first scout acquisition, and thus a first scout component 360 may be displayed within the radiation timeline 350 in line with the first examination phase icon 352 along the horizontal axis 390. The second examination phase may be a second scout acquisition, and thus a second scout component 362 may be displayed within the radiation timeline 350 in line with the second examination phase icon 354 along the horizontal axis 390. The first scout acquisition may be a head scan from a lateral view and the first scout component 360 that is displayed within the radiation timeline 350 may be a representation of the first scout acquisition. The second scout acquisition may be a head scan from an anterior-posterior (AP) view and the second scout component 362 that is displayed within the radiation timeline 350 may be a representation of the first scout acquisition. The first and second scout acquisitions, both being head scans, may have the same or nearly the same scan range 364. Therefore, the first and second scout components 360, 362 may be displayed at a shared range of coordinates along the vertical axis 392 as the scan range of z-coordinates for the two is the same (or nearly the same).

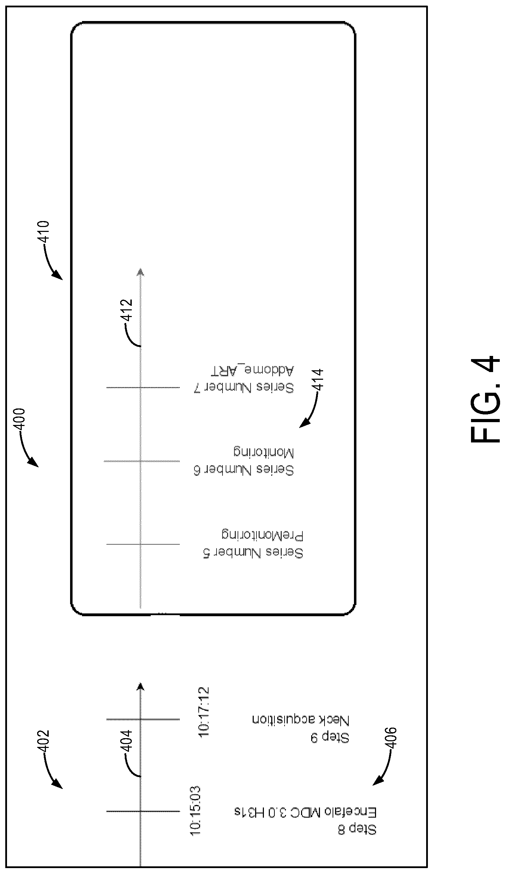

Turning now to FIG. 4, a portion of a timeline GUI 400 is shown. In some examples, as is shown in FIG. 4 and will be further described with respect to FIG. 10, more than one radiation timeline may be displayed within a timeline GUI. For example, a first radiation timeline 402 and a second radiation timeline 410 may be displayed within the timeline GUI 400 at the same time. The timeline GUI 400 may be displayed on a display of a care provider device (e.g., care provider device 134 of FIG. 1). Components and icons displayed within and/or on the radiation timelines may indicate or represent various phases of a selected imaging examination and radiation dose data thereof retrieved from a plurality of data repositories (e.g., PACS, imaging systems, etc.).

The first radiation timeline 402 may be similar to the radiation timelines described with respect to FIGS. 2-3B, wherein the first radiation timeline 402 includes a horizontal axis 404 and a vertical axis (not shown). One or more examination phases 406 may be plotted along the horizontal axis 404 as icons, each including a phase label and a timestamp as previously described. The first radiation timeline 402 may thus be a main or primary timeline, whereby examination phases with known timestamps (e.g., retrieved from one or more data repositories) are plotted chronologically, thereby depicting aspects of radiation dosage for phases of the selected imaging examination.

The second radiation timeline 410 may include a horizontal axis 412. The horizontal axis 412 may have one or more examination phases 414 plotted thereon. Each of the one or more examination phases 414 of the second radiation timeline 410, in contrast to the examination phases 406 of the first radiation timeline 402, may comprise an examination phase label but may not include a timestamp. Phases of the selected imaging examination that include timestamps may be plotted along the first radiation timeline 402 while phases that do not include timestamps may be plotted along the second radiation timeline 410. Thus, when segmenting data, for example according to rules stored in the rules module of FIG. 1, data may be segmented according to whether it has timestamps. In some examples, data without timestamps may be an example of data that has a missing data source, which may result in missing dose information. In other examples, data without timestamps may refer to data such as monitoring phases that do not have a specific timestamp. In some examples, however, monitoring phases may have known timestamps and as such may be included in the first radiation timeline 402. The secondary timeline that is displayed alongside or below the primary timeline within the timeline GUI may allow the user to see these data with missing dose information or otherwise without timestamps.

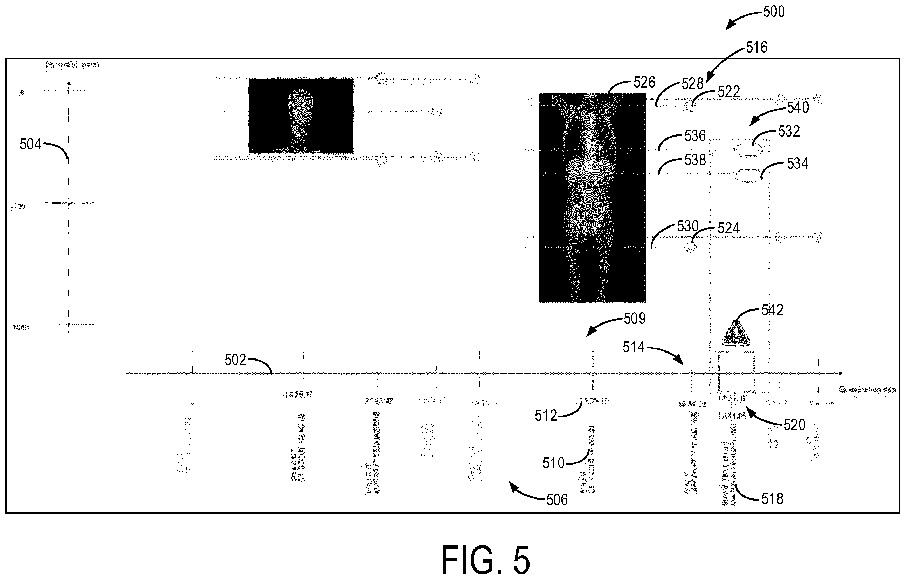

FIG. 5 shows another example radiation timeline 500. As described with respect to FIGS. 2-4, the radiation timeline 500 may be displayed within a GUI in response to user input, for example user selection of an imaging examination. The radiation timeline 500 may be displayed within a GUI, which may be displayed on a display of a care provider device (e.g., care provider device 134 of FIG. 1). Radiation timeline 500 may include a plurality of components, as will be further described below, that may be displayed individually or at the same time. Components and icons displayed within and/or on the radiation timeline 500 may indicate or represent various phases of the selected imaging examination and radiation dose data thereof retrieved from a plurality of data repositories (e.g., PACS, imaging systems, etc.).

The radiation timeline 500 may comprise a horizontal axis 502 on which one or more examination phases 506 are plotted. As described previously, the one or more examination phases 506 may each comprise a phase label and a timestamp. For example, a first examination phase 509 may comprise a phase label 510 and a timestamp 512. The radiation timeline 500 may further comprise a vertical axis 504. The vertical axis 504 may indicate z-coordinate of the patient that may correspond to scan ranges of scan acquisitions. Components plotted on the radiation timeline 500 may be aligned according to time along the horizontal axis 502 and according to scan range based on corresponding z-coordinate along the vertical axis 504.

As noted, the radiation timeline 500 may plot one or more components that correspond to one or more of the examination phases plotted along the horizontal axis 502. The one or more components may be scout acquisition components and/or sets of scan range components or scan range bars. For example, a first component 526 may correspond to a first examination phase 509. The first component 526 may be a scout acquisition component and may be a representation of the scout acquisition to which the first examination phase corresponds. A second component 516 may correspond to a second examination phase 514. The second examination phase 514 may be a series acquisition phase (e.g., an attenuation phase). In some examples, the second examination phase 514 may include an image acquisition that is within a similar scan range as the scout acquisition of the first examination phase. The second component 516 may be a set of scan range components or a scan range bar that comprises a top component 522 and a bottom component 524 (e.g., top edge and bottom edge in the case of a scan range bar). The top component 522 may correspond to an uppermost z-coordinate of the second examination phase's scan range and the bottom component 524 may correspond to a bottommost z-coordinate of the second examination phase's scan range. The scan range components (or scan range bars as is shown in FIGS. 10 and 11) may indicate the range of z-coordinates of the patient that were imaged in the corresponding examination phase, and therefore indicate to which area of the patient's body radiation was delivered during that particular examination phase.

In some examples, the top component 522 may include or be linked to a top projection line 528 that extends from the top component 522 across the first component 526 to indicate where the uppermost coordinate of the second examination phase lies in relation to the scout acquisition. Similarly, the bottom component 524 may include or be linked to a bottom projection line 530 that extends from the bottom component 524 to indicate where the bottommost coordinate of the second examination phase lies in relation to the scout acquisition. The distance between the top projection line 528 and the bottom projection line 530 may be the scan range of the second examination phase 514. The projection lines herein described may visually link components of image series acquisitions to a scout acquisition.

In some examples, more than one examination phase may correspond to a single plotted examination phase. For example, a plurality of series may be acquired in a sequential, consecutive manner with similar acquisition characteristics, such as description, protocol, scanning length, overall target region. In some examples, each of these acquisitions may have a shared scan range. Such acquisitions may be aggregated together and represented within the radiation timeline 500 together. For example, a third examination phase 518 may correspond to a plurality of series acquisitions. The third examination phase 518 may include a range of timestamps 520 represented on the horizontal axis 502 using a designated icon, such as a collated document icon with a displayed number of acquisitions included therein.

A third component 540 displayed within the radiation timeline 500 may correspond to the third examination phase 518. Similar to the second component 516, the third component 540 may comprise a top component 532 and a bottom component 534. The top component 532 may correspond to an uppermost z-coordinate and the bottom component 534 may correspond to a bottommost z-coordinate of the plurality of series acquisitions to which the third examination phase 518 corresponds. Similar to the second component 516, the top component 532 of the third component 540 may include a top projection line 536 that indicates visually where the uppermost z-coordinate lies with relation to the scout acquisition and a bottom projection line 538 that indicates visually where the bottommost z-coordinate lies with relation to the scout acquisition. The plurality of series acquisitions may be together represented by the third component 540 within the radiation timeline 500.

As discussed above, the data retrieved by the radiation dose management system from the data repositories may be processed to determine whether one or more criteria are met to trigger one or more alerts, such as alerts indicating over-irradiation, practice issues, or the like. For example, when an amount of radiation delivered to the patient is determined to exceed a preset threshold, an alert may be generated and displayed within the radiation timeline 500. For example, alert 542 may be displayed above the third examination phase 518 in response to detection that the third examination phase 518 includes a detected or otherwise known dosage radiation amount that exceeds a threshold amount, as based on a predetermined set of rules (e.g., rules of the rules module 124). As will be described further below, the alert 542 may be a selectable element with a GUI in which the radiation timeline 500 is displayed. When selected via a mouse click or hover, additional information about the alert and the dosage amounts of the corresponding examination phases may be shown in a pop-up window.

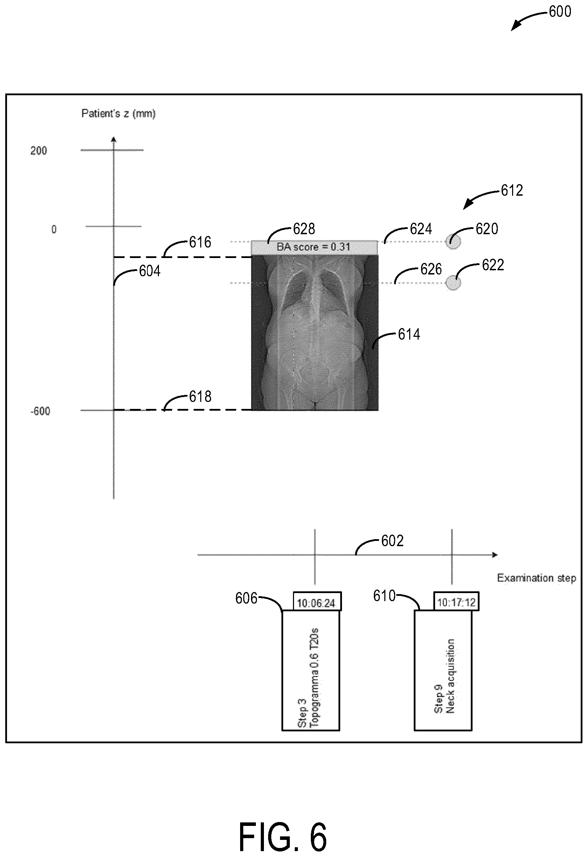

Turning now to FIG. 6, another example radiation timeline 600 is shown. As described with respect to FIGS. 2-5, the radiation timeline 600 may be displayed within a GUI in response to user input, for example user selection of an imaging examination. The radiation timeline 600, when included within a GUI, may be displayed on a display of a care provider device (e.g., care provider device 134 of FIG. 1). Radiation timeline 600 may include a plurality of components, as will be further described below, that may be displayed individually or at the same time. Components and icons displayed within and/or on the radiation timeline 600 may indicate or represent various phases of the selected imaging examination and radiation dose data thereof retrieved from a plurality of data repositories (e.g., PACS, imaging systems, etc.).

The radiation timeline 600, as previously described, may include a horizontal axis 602 that indicates time, increasing from left to right, and a vertical axis 604 that indicates z-coordinate of the patient being imaged, increasing from bottom to top (in the direction of the arrow). The radiation timeline 600 may plot one or more examination phases along the horizontal axis 602 and one or more components corresponding thereto. For example, a first examination phase 606 may be a scout acquisition and a scout component 614 may be displayed within the timeline according to the timestamp and the scan range. For example, a top edge 616 may be aligned with an uppermost z-coordinate of the scout acquisition and a bottom edge 618 may be aligned with a bottommost z-coordinate of the scout acquisition as plotted on the vertical axis 604.

A second examination phase 610 may also be plotted within the radiation timeline 600. In some examples, the second examination phase 610 may be an image acquisition that has a scan range that extends beyond the scan range of the scout acquisition (e.g., the first examination phase 606). As such, the image acquisition may be a blind acquisition, whereby at least of a portion of the scan range is obtained from z-coordinates not included in the scout acquisition. Similar to as described with respect to FIG. 5, a second component 612 may be included within the radiation timeline 600. The second component 612 may be a set of scan range components including a top component 620 and a bottom component 622. The top component 620 may include a top projection line 624 and the bottom component 622 may include a bottom projection line 626. The top component 620 may correspond to an uppermost z-coordinate of the scan range of the image acquisition and the bottom component 622 may correspond to a bottommost z-coordinate of the scan range. In other examples, the second component 612 may be a scan range bar wherein a top edge of the bar aligns with the uppermost z-coordinate and a bottom edge of the bar aligns with the bottommost z-coordinate.

In the example depicted in FIG. 6, the scout acquisition is a scout acquisition of a torso and the image acquisition may be of a neck of the patient. Thus, the uppermost z-coordinate of the scan range of the image acquisition may be above (e.g., a higher z-coordinate) the uppermost z-coordinate of the scout acquisition. In examples that include a blind acquisition, a blind acquisition element 628 may be selectively displayed within the radiation timeline 600. The blind acquisition element 628 may extend between the top edge 616 of the scout acquisition and the top projection line 624 that corresponds to the uppermost z-coordinate of the scan range of the image acquisition. The blind acquisition element 628 may thus visually indicate how much of the image acquisition is acquired blind in comparison to the scout acquisition. In some examples, a blind acquisition score may also be displayed within the blind acquisition element 628.

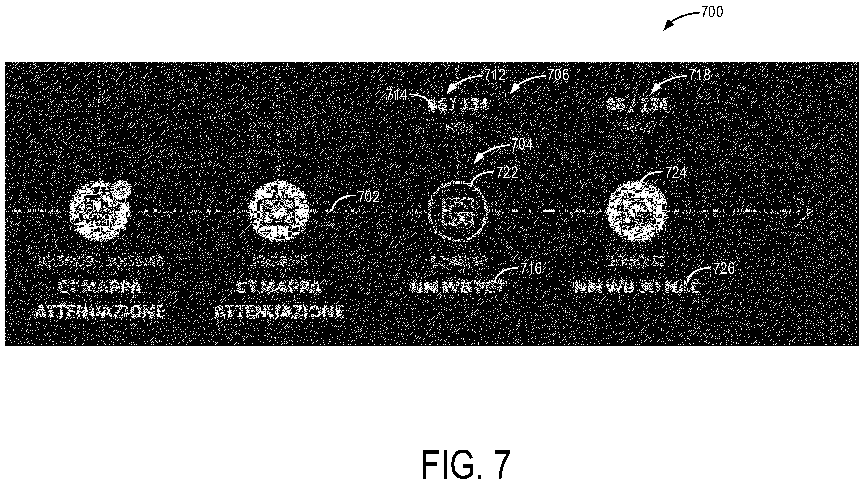

Turning now to FIG. 7, another portion of an example radiation timeline 700 is shown. As described with respect to FIGS. 2-3B and 5-6, the radiation timeline 700 may be displayed within a GUI in response to user input, for example user selection of an imaging examination. The GUI may be displayed on a display of a care provider device (e.g., care provider device 134 of FIG. 1). Radiation timeline 700 may include a plurality of components, as will be further described below, that may be displayed individually or at the same time. Components and icons displayed within and/or on the radiation timeline 700 may indicate or represent various phases of the selected imaging examination and radiation dose data thereof retrieved from a plurality of data repositories (e.g., PACS, imaging systems, etc.).

The radiation timeline 700 includes a horizontal axis 702, similar to the previous radiation timelines herein described. The horizontal axis 702 may indicate time from left to right and may have one or more examination phases 704 plotted thereon in chronological order. In examples in which the selected imaging examination includes a phase of administration of a radioactive isotope agent (e.g., 18-Fluorine), the radiation timeline 700 may also include isotope activity elements 706. The isotope activity elements 706 may be plotted with respect to the horizontal axis of the radiation timeline 700 such that components plotted on the isotope activity elements 706 are aligned with the examination phases plotted on the horizontal axis 702.

As an example, a first examination phase 716 may be plotted along the horizontal axis 702 at a first time, indicated by first icon722. A first activity element 712 of the isotope activity may correspond to the first time and may indicate a radioactivity level of the injected agent at the first time when the first examination phase 716 is performed. For example, upon initial administration, the radioactivity of the injected isotope may be 134 MBq and at the first time when the first examination phase 716 is performed, the radioactivity of the injected isotope, as indicated by the first activity element 712, may be 86 MBq. In some examples, an isotope activity level 714 may be displayed alongside the first activity element 712 indicating the amount of radioactivity at the corresponding time. In some examples, the isotope activity level 714 may also indicate the initial amount of radioactivity upon administration for comparison to the current amount.

In this way, the radioactivity of the isotope may be tracked as the imaging examination progresses through the phases thereof. For example, the user may see that at the first time 722, the radioactivity is a first amount and at a second time indicated by second icon 724, when a second examination phase 726 occurs, the radioactivity of the isotope may be a second amount, as indicated by a second radioactivity level 718. The second amount may be less than or equal to the first amount and the user may easily visualize the progression of radioactivity throughout the imaging examination.

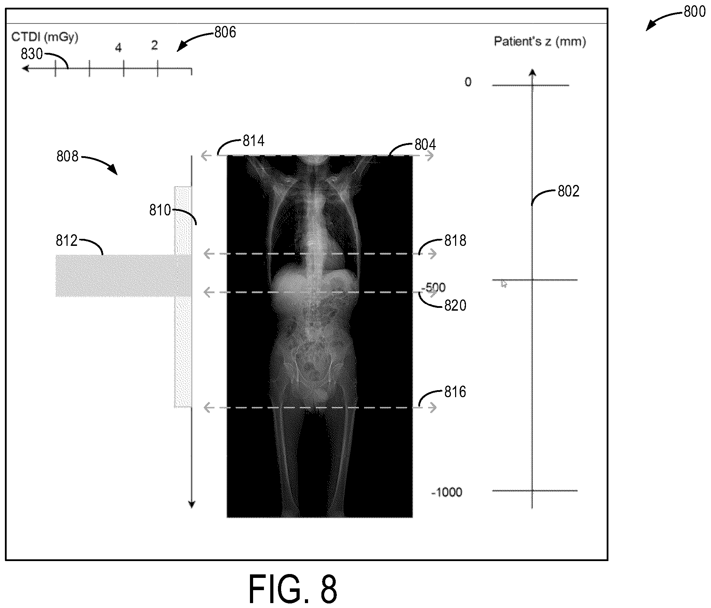

Turning now to FIG. 8, an example component of a radiation timeline is shown. As described with respect to FIGS. 2-7, the radiation timeline may be displayed within a GUI in response to user input, for example user selection of an imaging examination. The radiation timeline, when included within a GUI, may be displayed on a display of a care provider device (e.g., care provider device 134 of FIG. 1). The radiation timeline may include a plurality of components, such as component 800 herein described plotted with respect to time and z-coordinate. Components and icons displayed within and/or on the radiation timeline may indicate or represent various phases of the selected imaging examination and radiation dose data thereof retrieved from a plurality of data repositories (e.g., PACS, imaging systems, etc.).

As noted, the component 800 may be plotted with respect to time (e.g., as on a horizontal axis) and scan range of z-coordinates as shown on a vertical axis 802. The component 800 may correspond to an examination phase, such as a torso scout acquisition and the component 800 may be a representation 804 of the examination phase (e.g., the scout acquisition may be displayed within the timeline as a size relative to the vertical axis 802). As described previously, a top edge of the representation 804 may align with an uppermost z-coordinate of the acquisition and a bottom edge of the representation 804 may align with a bottommost z-coordinate of the acquisition. The representation of the scout acquisition may thus be proportionally sized within the constraints of the scan range thereof.

The component 800 may further include, in some examples, a radiation dose element 806. The radiation dose element 806 may comprise a plurality of dosage bars 808 indicating an amount of irradiation to different areas of the patient, in some instances those areas may correspond to z-coordinates within the scan range of the scout acquisition. The radiation dose element 806 may be plotted against a radiation dose axis 830 and the vertical axis 802. The radiation dose axis 830 indicates amount of radiation (e.g., CT dose index) in milligray (mGy) or other suitable unit, increasing from right to left.

The plurality of dosage bars 808 may be displayed relative to the representation 804 such that each of the bars corresponds to an area of the patient's body as shown in the representation 804. For example, a first dosage bar 810 may extend from a first z-coordinate 814 to a second z-coordinate 816. The first dosage bar 810 may correspond to one or more image series acquisitions obtained within a first scan range from the first z-coordinate 814 to the second z-coordinate 816. Similarly, a second dosage bar 812 may extend from a third z-coordinate 818 to a fourth z-coordinate 820. The second dosage bar 812 may correspond to one or more image series acquisitions obtained within a second scan range from the third z-coordinate 818 to the fourth z-coordinate 820. The first dosage bar 810 may correspond to an amount of radiation of approximately 1 mGy while the second dosage bar 812 may correspond to an amount of radiation of approximately 8 mGy. The dosage bars 808 may be cumulative, for example, the second dosage bar 812 includes radiation information from the first z-coordinate 814 to the second z-coordinate 816 (e.g., 1 mGy) and from the third z-coordinate 818 to the fourth z-coordinate 820 (e.g., 7 mGy). As such the one or more image series acquisitions obtained within the second scan range may have a higher CT dose index than the one or more image series acquisitions obtained within the first scan range.

The radiation dose element 806 may be displayed alongside scout acquisition components within the radiation timeline. Further, as will be described with respect to FIGS. 10 and 11, the dosage bars of the radiation dose element 806 may correspond to sets of scan range elements of examination phases such that the user may identify amounts of irradiation delivered during each examination phase.

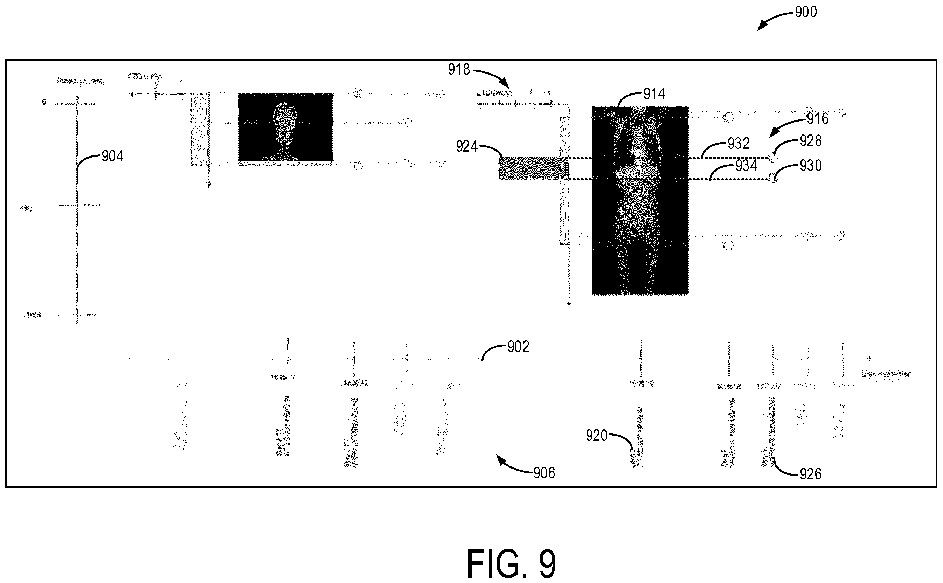

Turning now to FIG. 9, an example radiation timeline 900 is shown. As described with respect to FIGS. 2-8, the radiation timeline 900 may be displayed within a GUI in response to user input, for example user selection of an imaging examination. The GUI may be displayed on a display of a care provider device (e.g., care provider device 134 of FIG. 1). Radiation timeline 900 may include a plurality of components plotted with respect to time and z-coordinate. Components and icons displayed within and/or on the radiation timeline may indicate or represent various phases of the selected imaging examination and radiation dose data thereof retrieved from a plurality of data repositories (e.g., PACS, imaging systems, etc.).

The radiation timeline 900 may include a horizontal axis 902 that indicates time from left to right and a vertical axis 904 that indicates z-coordinates increasing from bottom to top. The horizontal axis 902 may have one or more examination phases 906 plotted thereon, each including a phase label and a timestamp as previously described. Each of the one or more examination phases 906 may correspond to one or more components displayed within the radiation timeline 900. For example, a first examination phase 920 may be plotted on the horizontal axis 902. In some examples, the first examination phase 920 may be a scout acquisition of a body (e.g., excluding head). A first component 914 may correspond to the first examination phase 920. In examples in which the first examination phase 920 is a scout acquisition, the first component 914 may be a representation of the scout acquisition, for example a proportional image of the scout acquisition displayed in the timeline aligned with the vertical axis 904 based on the z-coordinates of the scan range of the scout acquisition. The first component 914 may be displayed in line with the first examination phase 920.

The radiation timeline 900 may further include one or more sets of scan range components, such as set 916. The set 916 may correspond to a second examination phase 926 plotted along the horizontal axis 902. The second examination phase 926, in some examples, may be an image series acquisition phase. The set 916 may comprise a top component 928 and a bottom component 930, similar to as previously described. The top component 928 may correspond to an upper most z-coordinate and the bottom component 930 may correspond to a bottom most z-coordinate of the image series acquisition to which the set 916 corresponds.

The radiation timeline 900 may further include a radiation dose element 918. As described with respect to FIG. 8, the radiation dose element 918 may include a radiation dose axis indicating an amount of irradiation delivered to the patient and a plurality of dosage bars that are plotted according to the radiation dose axis and the vertical axis 904 of the radiation timeline 900. As such, each of the plurality of dosage bars may correspond to scan ranges of z-coordinates. In some examples, the dosage bars may directly correspond to sets of scan range components plotted within the radiation timeline 900. As an example, dosage bar 924 of the radiation dose element 918 may correspond to an accumulation of radiation from the set 916 and others that overlap with the corresponding scan range, whereby a scan range of the dosage bar 924 is the same as the scan range of the set 916. For example, an upper most z-coordinate of the scan range of the dosage bar 924 may be the uppermost z-coordinate indicated by the top component 928. First z-coordinate line 932 and second z-coordinate line 934 may indicate the uppermost and bottommost z-coordinates of the shared scan range. As an example, the scan range indicated by the first and second z-coordinate lines 932, 934 may correspond to the patient's mid torso, surrounding the diaphragm. The image series acquisition that the set 916 corresponds to may include a plurality of series acquisitions imaging the patient's mid torso. In such examples, the dosage bar 924 may be the largest or one of the largest dosage bars of the radiation dose element 918 as a result of a higher amount of irradiation being applied to that scan range than to other areas of the patient's body.

While one scout acquisition and corresponding component has been described herein, it should be appreciated that more than one scout acquisition may be performed during an imaging examination. As is shown in FIG. 9, a first scout acquisition may be performed of the head and a second scout acquisition may be performed of the torso. In many imaging studies, image acquisitions may be performed subsequent to the first scout acquisition and subsequent to the second scout acquisition. In the radiation timeline as herein presented, components corresponding to examination phases (e.g., image acquisitions) performed after the first scout acquisition but before the second scout acquisition may be displayed within the timeline as connected to the first scout acquisition. In some examples, components may be matched to a corresponding scout acquisition and linking lines may be displayed between the representation of a scout acquisition and a corresponding component. For example, components corresponding to phases performed after the second scout acquisition may be displayed within the timeline as connected to either the first or second scout acquisition depending on scan range.

Turning now to FIG. 10, an example GUI 1000 is shown that displays one or more radiation timelines (e.g., timeline 106) generated by radiation dose management system 102 of patient information system 100. The radiation timelines described with respect to FIGS. 2-9 may be examples of the one or more radiation timelines that may be displayed within the GUI 1000. In some examples, the GUI 1000 may display more than one radiation timeline, for example when data retrieved by the radiation dose management system 102 includes both data with timestamps and data without timestamps. Data points and icons within each of the radiation timelines may indicate or represent examination phases and data thereof retrieved from a plurality of data repositories (e.g., one or more imaging systems, PACS, RIS, etc.). The GUI 1000 may be accessed via a selectable link within one or more EMRs or may be a standalone application (e.g., the radiation dose management system 102 of FIG. 1) accessible by the computing device. The one or more radiation timelines that are displayed within the GUI 1000 may be displayed in response to user selection of an imaging examination and may thus be specific to the selected imaging examination. The radiation dose management system 102 by which information displayed within the timeline GUI 1000 is generated may be patient centric, such that a link within an EMR may be specific to a patient and selection of the link may trigger display of information within the GUI 1000 relevant to the patient. It should be understood that while the GUI 1000, and other GUIs herein described, is described with respect to a PET-CT imaging examination, other imaging modalities, including modalities that use irradiation and modalities, such as MRI, that do not use irradiation, may also be displayed in similar timelines within the GUI 1000 without departing from the scope of this disclosure.

The GUI 1000 may display a first radiation timeline 1002. The first radiation timeline 1002, similar to the other radiation timelines described above, may include a horizontal axis 1004 that plots time in a non-linear manner increasing from left to right. One or more examination phases 1006 may be plotted along the horizontal axis 1004 according to timestamps associated therewith, whereby examination phases occurring earlier are placed leftwards on the horizontal axis and phases occurring later are placed relatively rightward on the horizontal axis. Each of the one or more examination phases 1006 may be represented by an icon displayed along the horizontal axis 1004. For example, a first examination phase may be displayed along the horizontal axis 1004 by a first icon 1008 and a second examination phase may be displayed along the horizontal axis 1004 by a second icon 1012.

In some examples, information regarding each of the one or more examination phases 1006 may be displayed below, above, within, or otherwise near a corresponding icon. The information may include an examination phase title or label and a timestamp. Alternatively or additionally, each of the icons, such as first icon 1008 and second icon 1012, may be selectable elements that when selected via user input or hovered over (e.g., hovered over with a cursor), may display a pop up window overlaid on the first timeline 1002 without the GUI 1000 that displays the label and timestamp of the corresponding examination phase as well as, in some examples, other information and/or selectable links as will be further described.

As an example, third icon 1018 displayed along the horizontal axis 1004 corresponds to a third examination phase. When hovered over or selected, a pop-up window 1028 may be displayed within the first timeline 1002. The pop-up window 1028 may display additional information regarding the third examination phase, including, as non-limiting examples, that the third examination phase was a partially blind acquisition, a protocol name, a mean CT dose index, a series number, a target region, and a scanning length. As another example, a pop-up window corresponding to a contrast agent injection phase may include information such as amount of injected contrast, type of contrast, and the like. In this way, multiple types of patient data relating to the imaging examination may be displayed at the same time to the user, such as both nuclear medicine data (e.g., radioactive agent injections) and CT-based data (e.g., iodine contrast agent injections).

In other examples, the pop-up window 1028 may include a selectable link to a PACS that when selected launches a separate window of the PACS displaying the corresponding acquisition of the third examination phase for the user to view. In this way, the GUI and radiation dose management system may provide a convenient method for visualizing radiation dose data of an imaging study and allowing the user to visualize corresponding imaging series by linking to one or more data repositories.

As previously described, each of the one or more examination phases 1006 may correspond to a component or element within the first radiation timeline 1002. Different types of examination phases may correspond to different types of components. For example, the first examination phase may be a torso scout acquisition and thus a corresponding first component 1010 may be a representation of the scout acquisition. The first component 1010 may be displayed within the first radiation timeline 1002 along the horizontal axis 1004 according to the timestamp of the first examination phase and along a vertical axis 1003 according to a scan range of from the scout acquisition. The vertical axis 1003 may indicate z-coordinate increasing bottom to top. Components may be displayed within the first radiation timeline 1002 based on scan range of z-coordinates aligned with corresponding z-coordinates of the vertical axis 1003, as previously described.

As another example, the second examination phase may be an image series acquisition and thus a corresponding second component 1014 may be a scan range bar. The second component 1014 may be displayed within the first radiation timeline 1002 along the horizontal axis 1004 according to the timestamp of the second examination phase and along the vertical axis 1003 according to the scan range of the image series acquisition such that a top edge of the bar aligns with an uppermost z-coordinate and a bottom edge of the bar aligns with a bottommost z-coordinate. In some examples, the first examination phase may be linked to the second examination phase, for example when the image series acquisition is performed based on the scout acquisition, and thus the second component 1014 may be linked to the first component 1010 by projection lines 1040. Similarly, in some examples, other examination phases may be linked to the scout acquisition as well and multiple projection lines may be included in the first radiation timeline 1002 connecting each corresponding component to the first component 1010. The projection lines 1040, similar to as previously described, may indicate the uppermost z-coordinate and bottommost z-coordinate of the image series acquisition. The scan range bar, as previously noted, may thus indicate to which portion of the patient's body radiation was delivered during the corresponding examination phase.

Further, the first component 1010, when the first component 1010 is a representation of a scout acquisition that is linked to one or more image series acquisitions, may be linked to a dose accumulation element 1016. As described with respect to FIG. 8, the dose accumulation element may include one or more dosage bars aligned along a radiation dose axis and the vertical axis 1003. The dose accumulation element 1016 may thus visually indicate an amount of irradiation to each area of the patient's body based on the radiation dose data of each of the irradiating examination phases and the scan ranges thereof. In this way, the user may easily visualize accumulated radiation doses, which may help to inform further clinical decisions.

In some examples, the GUI 1000 may include one or more selectable elements that allow the user to customize content displayed within the GUI 1000. For example, the dose accumulation element 1016 may be associated with selector elements 1042 that allow the user to toggle between different types of radiation measurement, such as CT dose index (CTDI) and size specific dose estimate (SSDE). The user may choose a type of radiation measurement that is available based on the imaging modality, patient body size, or other factors. The one or more selectable elements may also include a first element 1044 and a second element 1046. The first element 1044, in some examples, may allow the user to toggle on/off display of all scan range projections (e.g., projection lines) and the second element 1046, in some examples, may allow the user to toggle on/off display of blind acquisition elements. Alternatively or additionally, the user may select multiple scan range elements (e.g., scan range bars) to display scan range projections thereof.

For example, when the second element 1046 is toggled to on, as shown in FIG. 10, one or more blind acquisition elements may be displayed within the first radiation timeline 1002. For example, a fourth examination phase, represented by fourth icon 1050 along the horizontal axis 1004, which may be a second series acquisition, may be a blind acquisition wherein a portion of the scan range thereof lies outside the scan range of the linked scout acquisition. As such, a corresponding third component 1020 may include a first portion 1024 and a second portion 1022. The first portion 1024 may correspond to a first portion of the scan range of the second series acquisition that lies within the scan range of the scout acquisition and the second portion 1022 may correspond to a second portion of the scan range of the second series acquisition that lies outside the scan range of the scout acquisition. A blind acquisition element 1026 may also be displayed within the first radiation timeline 1002. As described with respect to FIG. 6, the blind acquisition element 1026 may be displayed directly above or directly below the first component 1010. The blind acquisition element 1026 may be aligned along the vertical axis 1003 according to the z-coordinates of the second portion of the scan range of the second series acquisition and may thus indicate how far outside the scan range of the scout acquisition the second series acquisition lies.

Further, when an examination phase includes a blind acquisition, an alert may be displayed within the icon of the corresponding phase icon. For example, the fourth icon 1050 representing the fourth examination phase may include an alert 1052 indicating to the user that a practice issue, such as a partially blind acquisition, is included in the third examination phase.

In addition to the first radiation timeline 1002, the GUI 1000 displays a second radiation timeline 1030. The second radiation timeline 1030 may be included within the GUI 1000 when data retrieved from the one or more data repositories includes data without timestamps. The second radiation timeline 1030 may not be time aligned and thus may not correspond to the horizontal axis 1004 or the vertical axis 1003 of the first radiation timeline 1002. The second radiation timeline 1030 may plot one or more examination phases that are not timestamped, such as pre-monitoring phases, monitoring phases, and the like. The non-timestamped examination phases may be sorted along the second radiation timeline 1030 by series number. In this way, the GUI 1000 may display both timestamped radiation data and non-timestamped radiation data alongside each other to allow the user to visualize both at the same time to understand the full scope of the selected imaging examination.

In some examples, the first radiation timeline 1002 may be displayed within the GUI 1000 in its entirety such that each component is displayed within the GUI 1000 at the same time. As previously described, the examination phases plotted along the horizontal axis of the first radiation timeline 1002 may be arranged chronologically in a non-linear manner. The radiation dose management system may be configured to arrange the examination phases based on the associated components to be displayed within the timeline such that all components may be visualized at the same time within the window of the GUI 1000. Further, the system may be configured to resize the elements proportionally when the window of the GUI 1000 is resized. For example, the GUI 1000 may be accessed via an application that is displayed on a care provider device within a designated window. The window may be resized manually by the user and the first radiation timelines, and the other radiation timelines herein described, may be tied to the aspect ratio of the window of the GUI 1000 such that the components therein are displayed proportionally and all components are displayed at the same time, allowing the user to see all examination phases and components within the timelines even when there is limited display space within the GUI window.

In some examples, the amount of examination phases of the selected imaging examination may exceed the available space within the radiation timeline(s) for a given window of the GUI 1000. As noted, the radiation timeline may be tied to the aspect ratio of the GUI window. When the amount of phases exceeds a threshold for display within the window in a proportional manner, the user may horizontally scroll along the GUI to scroll through the examination phases and corresponding components and/or elements along the horizontal axis without altering other elements within the GUI 1000. In this way, the radiation dose management system and graphical timelines herein described may allow the user to easily see phases of the selected imaging examination all at the same time without having to sort through multiple entries of tabular data.

Turning now to FIG. 11, an example GUI 1100 is shown that displays one or more radiation timelines (e.g., timeline 106) generated by radiation dose management system 102 of patient information system 100. The radiation timelines described with respect to FIGS. 2-3B and 5-8 may be examples of the one or more radiation timelines that may be displayed within the GUI 1100. In some examples, the GUI 1100 may display more than one radiation timeline, for example when data retrieved by the radiation dose management system 102 includes both data with timestamps and data without timestamps. Data points and icons within each of the radiation timelines may indicate or represent examination phases and data thereof retrieved from a plurality of data repositories (e.g., one or more imaging systems, PACS, RIS, etc.). The GUI 1100 may be accessed via a selectable link within one or more EMRs or may be a standalone application accessible by the computing device. The one or more radiation timelines that are displayed within the GUI 1100 may be displayed in response to user selection of an imaging examination and may thus be specific to the selected imaging examination. The radiation dose management system 102 by which information displayed within the timeline GUI 1100 is generated may be patient centric, such that a link within an EMR may be specific to a patient and selection of the link may trigger display of information within the GUI 1100 relevant to the patient. It should be understood that while the GUI 1100, and other GUIs herein described, is described with respect to a PET-CT imaging examination, other imaging modalities, including modalities that use irradiation and modalities, such as MRI, that do not use irradiation, may also be displayed in similar timelines within the GUI 1100 without departing from the scope of this disclosure.

The GUI 1100, similar to the GUI 1000 described above, may include a first radiation timeline 1102 that includes a horizontal axis 1104 and a vertical axis 1105. The horizontal axis 1104 may indicate time increasing from left to right, time plotted non-linearly thereon. The vertical axis 1105 may indicate z-coordinate, increasing from bottom to top. One or more examination phases 1106 of the selected imaging examination may be plotted along the horizontal axis 1104 in chronological order from left to right. Each of the one or more examination phases 1106 may be plotted on the horizontal axis 1104 with a representative icon. An examination phase label or title and a timestamp may also be displayed within the GUI 1100 for each of the plotted one or more examination phases 1106.

The GUI 1100 may display one or more components 1108 within the first radiation timeline 1102. Similar as described above, each of the one or more components 1108 may correspond to one of the one or more examination phases 1106 plotted along the horizontal axis 1104. As such, each component may be aligned along the horizontal axis 1104 according to its corresponding timestamp. Each component may be aligned along the vertical axis 1105 according to the corresponding examination phase's scan range, as previously described. The one or more components may comprise representations of scout acquisitions, scan range bars or sets of scan range components, and blind acquisition elements. Further, the first radiation timeline 1102 may display one or more alerts, for example an over irradiation alert when radiation data from the one or more examination phases 1106 meets or exceeds a preset irradiation amount threshold stored in memory.

The GUI may also display isotope activity elements 1110 in examples in which the selected imaging examination includes injection of a radioactive agent, such as an imaging study that includes PET with injection of FDG or other agent. The isotope activity elements 1110, in some examples, may be displayed within or overlaid on the first radiation timeline 1102 such that the horizontal axis 1104 of the first radiation timeline 1102 is the horizontal axis of the isotope activity elements 1110. The isotope activity elements 1110, as described with respect to FIG. 7, may display an amount of radioactivity of the injected radioactive agent at one or more of the one or more examination phases 1106. In some examples, an amount of radioactivity for each data point plotted of the isotope activity elements 1110 may be textually displayed along with each plotted data point.

As an example, a first examination phase 1114 may be plotted as an icon along the horizontal axis 1104. The first examination phase 1114 may have a timestamp as retrieved from the one or more data repositories and may be plotted along the horizontal axis 1104 according to the timestamp. The isotope activity elements 1110 may include a corresponding first data point 1112. The first data point 1112 may be plotted of the isotope activity elements aligned with the timestamp of the first examination phase 1114. Further, the first data point 1112 may be plotted with a corresponding radioactivity level displayed textually within the first radiation timeline 1102.

In some examples, each of the data points of the isotope activity elements 1110 may be selectable elements that when hovered over launch a pop-up window as an overlay on the first radiation timeline 1102 and/or the isotope activity elements 1110. For example, second data point 1116 of the isotope activity elements 1110 may be a selectable element that when hovered over triggers display of a pop-up window. The pop-up window may include information such as units of radioactivity (e.g., MBq), amount of radioactivity if not displayed for the isotope activity elements, an amount of radioactive decay for the given injected radioactive agent, and/or the like.

The horizontal axis 1104 of the first radiation timeline 1102, and other x-axes herein described, may indicate time in a non-linear manner such as to allow each of the one or more examination phases and corresponding components to be visualized in a single GUI window. Further, resizing of the GUI 1100 may result in the first radiation timeline 1102 and the isotope activity elements 1110 being resized proportionally with respect to the aspect ratio defined within the GUI 1100. As such, components displayed within the timelines, for example the representations of the scout acquisitions and placements of examination phases along the x-axes may be adjusted and/or initially displayed relative to the size of the GUI 1100 such that components are displayed in full and with proportional dimensions. In this way, despite limited display space within the GUI 1100, the graphical timelines herein disclosed may present radiation dose data in a graphical manner that allows the user to see examination phases and associated radiation dose information thereof.

In the event of a GUI window with horizontal dimensions too small to display all of the one or more examination phases and corresponding components fully within the GUI window based on aspect ratio constraints, a horizontal scroll bar may be included within the GUI so as to allow the user to scroll horizontally to adjust a window of the horizontal axis 1104 that is currently displayed. In this way, the radiation timelines and GUIs herein described may allow the user to easily visualize each phase of a given imaging study with relevant radiation accumulation at various scan ranges and radioactivity information all at once within a limited display space.

Further, the components that are displayed within a radiation timeline may be updatable to the system in an evolutive manner. For example, a first set of component types may be stored in memory and displayed corresponding to the selected imaging study. New components may be added to memory such that in a subsequent session or usage, the new component(s), if applicable, may be displayed within the radiation timeline for the same selected imaging study or different selected imaging studies. In this way, the system herein provides flexibility for ever changing modalities, acquisition types, and the like.

The GUIs herein described may be one example GUI page of a radiation dose management application. The radiation dose management application may include a first GUI that is launched in response to opening of the radiation dose management application. In some examples, the first GUI may include a list of patients for which radiation dose management data is available and user selection of a specific patient may launch a second GUI that lists each imaging examination for the patient. In other examples, the first GUI may include the list of each imaging examination for a patient in examples in which the radiation dose management system is launched specific to the patient (e.g., via selection of a link within an EMR for the patient). User selection of one of the imaging examinations within the list may launch a GUI that displays a radiation timeline, such as GUI 1000 or GUI 1100 as described above, and thus the data displayed within the radiation timeline may be specific to the selected imaging examination.

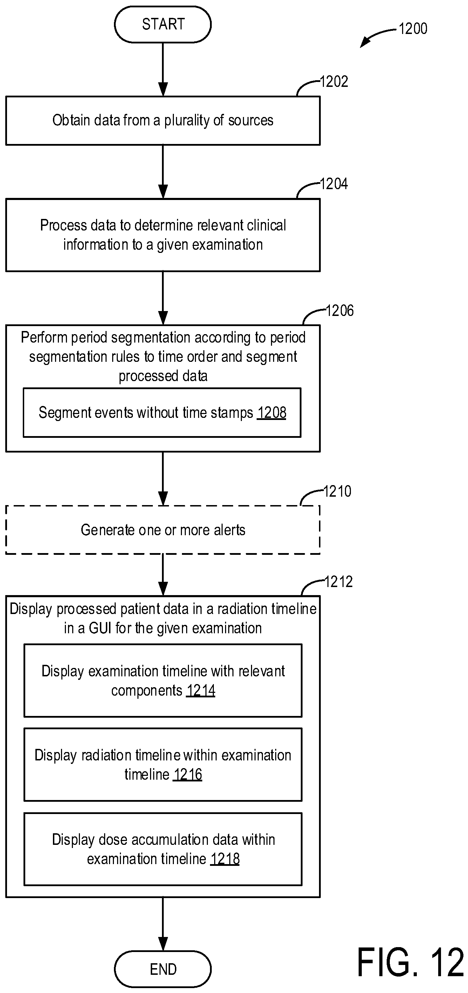

Turning now to FIG. 12, a flowchart illustrating a method 1200 for generating and displaying a radiation timeline within a GUI is shown. Method 1200 may be carried out according to instructions stored in memory of a computing device (e.g., memory 130 of radiation dose management system 102 of FIG. 1), which may be executed by a processor of the computing device (e.g., processor(s) 132). While method 1200 is described specific to one imaging examination of one patient, at least portions of method 1200 may be performed simultaneously for a plurality of imaging examinations and/or a plurality of patients.

At 1202, patient data from a plurality of sources is obtained for the patient. The patient data may be obtained from EMR databases, PACS, RIS, and/or imaging systems including one or more of CT systems, PET systems, MRI systems, multi-modality systems such as PET-CT systems, and the like, as described previously with respect to FIG. 1. The patient data may include imaging examination data, such as examination protocol, series information, phase timestamps, and corresponding radiation dose data. The patient data may additionally comprise images or representations of images obtained during the examination, such as scout acquisition images, for example from PACS. Examination phases may include series acquisitions, scout acquisitions, radioactive agent injections, contrast agent (iodine) injections, and the like. As an example, the imaging examination data may include one or more examination phases for each study, including phase labels and timestamps thereof, and radiation dose data for one or more of the one or more examination phases. The radiation dose data may include an amount of irradiation delivered to the patient, scan range for which the irradiation was delivered, one or more isotope activity levels in the case of nuclear medicine studies that include injection of a radioactive agent, and the like. In some examples, the patient data may be obtained in response to a request (e.g., a user input or selection) to display a radiation timeline for the imaging examination. In other examples, the patient data may be obtained in response to a request to display a radiation dose management application. For example, when viewing an EMR for the patient, a user may select a link displayed as part of the EMR interface in order to initiate retrieval of imaging examination and radiation dose data for the patient. Alternatively, within the radiation dose management application, a user may select an imaging examination from a list which may initiate retrieval of the data.

At 1204, the patient data is processed to determine relevant clinical information. In some examples, patient data obtained from the one or more sources may include a plurality of data types for a plurality of patients and/or for a plurality of imaging examinations. Thus, processing the patient data may process out data that is irrelevant to radiation dose management, such as demographic data, family history data, and the like. In some examples, processing may include parsing out patient data that is relevant, such as patient history data that would result in lower tolerances to irradiation. In some examples, processing the patient data may include filtering the data in response to user selection of the imaging examination such that only data specific to the imaging examination is considered. For example, the radiation dose management system, as a standalone application, may include an initial start page with a drop-down menu through which the user may select a patient and an imaging examination they wish to view radiation dose data of.

At 1206, period segmentation is performed on the processed patient data according to period segmentation rules to time order and segment events. The period segmentation rules may be included in the rules module of the radiation dose management system. The patient data processed at 1204 may include multiple parameters of data, including examination phases classifications, scout acquisition scan ranges and images thereof, image series acquisition scan ranges, timestamps, isotope activity levels, delivered irradiation data, and the like. Data may be segmented into parameters and types; the data therefore being grouped. For example, for a multi-modality imaging study, such as a PET-CT scan, examination phases may be segmented based on which modality they belong to. As a result, data may be presented differently depending on which group it is segmented into. For examples, in a displayed timeline, data points corresponding to CT phases of a PET-CT study may be displayed with a first icon type or color and data points corresponding to PET phases of the PET-CT study may be displayed with a second icon type or color different than the first icon type or color to allow the user to easily differentiate the various phases and their individual effect on cumulative irradiation.

Further, as described, one or more of the examination phases may have associated timestamps that identify date, hour, minute, and in some cases second in which they were executed. Examination phases may ultimately be plotted within a timeline according to an earliest registered timestamp, for example a series acquisition may start at a first time and end at a second time. The first time thereof may be used as the timestamp to plot the series acquisition along the horizontal axis of the timeline. Period segmentation may time order the data with timestamps chronologically such that presentation of the data (e.g., display of the data within a radiation timeline) may be arranged chronologically. Additionally, in some examples, one or more of the examination phases may not have associated timestamps, as previously discussed. In such examples, events without timestamps may be segmented from events with timestamps, as noted at 1208. In this way, events without timestamps, which may not have a place on a chronologically presented timeline, may be presented on their own within the same GUI, for example within a secondary, timeless graph or timeline.

At 1210, one or more alerts are generated based on the processed patient data. As discussed, processing the patient data may include applying a set of rules stored in memory. In some examples, one or more rules may relate to amounts of irradiation, both cumulatively or at a single time, various practice standards or recommendations, and the like. If the obtained data meets criteria for one or more alerts, for example an over-irradiation alert, the one or more alerts may be generated. If the obtained data does not meet any criteria for alerts stored in the rules module, no alerts may be generated.

At 1212, the processed patient data is displayed in one or more radiation timelines within a GUI. The GUI (e.g., GUI 1000 of FIG. 10) may be displayed on a display of a care provider device (e.g., care provider device 134 of FIG. 1). The GUI may include one or more radiation timelines, for example a first radiation timeline for data with timestamps, a second radiation timeline for data without timestamps, and a third timeline of isotope activity. The radiation timelines, when displaying data with timestamps, may display one or more examination phases of the given imaging examination along a horizontal axis. Corresponding components may be generated based on the patient data and may be displayed within the one or more radiation timelines. As described above, the components may include representations of scout acquisitions for scout acquisition examination phases and scan range components (e.g., sets of scan range components or scan range bars) for image series acquisitions. Further, components may be linked to each other, for example a scan range component of an acquisition may be linked to a representation of a scout acquisition. The linkage may be displayed within the radiation timeline with projection lines that indicate upper and lower borders of scan ranges. Further, any generated alerts may be displayed as alert elements within the one or more radiation timelines. Further still, display of one or more radiation timelines may be linked to an aspect ratio of the window of the GUI. In this way, sizes and dimensions of components displayed within the one or more radiation timelines and placements of the examination phases along the horizontal axis of the one or more radiation timelines may be based on the size of the GUI window, the aspect ratio, and the radiation data and imaging examination data that is to be displayed therein.

As describe with respect to FIGS. 10 and 11, the displayed GUI may be interacted with by the user in one or more ways, including modifying the GUI to display pop-up windows, manipulating the data displayed by toggling elements on and off, and more. The GUI and the timelines therein may thus be flexible to fit the user's preferences of what information they wish to visualize and how.

As previously described, the examination phases and corresponding components may be displayed in a non-linear chronological manner. As such, the examination phases may be ordered chronologically along the timeline, however the phases may not be placed along the horizontal axis proportionally with respect to time. As a non-limiting example, a first examination phase may be performed at a first time, a second examination phase may be performed at a second time 3 seconds after the first time, and a third examination phase may be performed at a third time, 10 minutes after the second time. Icons corresponding to the first and second examination phases may be placed a first distance apart and the icon corresponding to the third examination phase may be placed a second distance from the icon corresponding to the second examination phase. The first and second distances may be determined based on configured size of corresponding components to be displayed within the GUI (e.g., based on window size, number of examination phases, size of corresponding components, etc.) and may not be proportional to the differences in timestamps of the first, second, and third examination phases. Because of this, each examination phase and components thereof may be displayed with adequate space for the user to see each element of the timeline without having any overlapping elements or covered elements as would occur with a linear timeline.

The technical effect of presenting radiation data in a timeline in a GUI as disclosed herein is that user/clinician time spent searching individually reported tabular radiation data for an imaging study is drastically reduced. As tabular data reports each series, acquisition, or other examination phase separately, the user spends time sorting through a multitude of entries to find relevant information. The radiation timelines herein presented provide a visual for the user to view each phase and relevant data thereof all in one place, including dose accumulation information and potential issues in the form of alerts, thereby reducing cognitive overload and overall time spent for the user.

Further, the radiation timelines herein present aggregated radiation data for multi-modality studies that would previously be reported in separate pages of tabular data. As an example, for a PET-CT study that includes examination phases relating both to nuclear medicine and to x-ray (e.g., CT), previous radiation dose management systems report radiation data for phases thereof, such as CT acquisition phases, iodine contrast injection phases, PET phases, and radioactive agent injection phases, in separate windows, tabs, or tables, causing the user to have to switch back and forth to gather a full understanding of the imaging study. The radiation timeline herein presented aggregates such data into one timeline where the exam phases are presented in a time ordered fashion such that the user may easily visualize data of the phases as it actually progresses during the study. When the radiation timeline is displayed within the GUI on the display device and when the additional display elements (e.g., the various pop-up windows launched in response to user input to a selectable element) are displayed, the one or more data repositories (e.g., imaging systems, PACS, EMRs) may exist in an un-launched state (e.g., not displayed and not currently accessed by the display device or care provider device). In this way, the data from the data repositories may be used to generate the radiation timeline and elements thereof. The patient data (e.g., examination phases, timestamps, radiation dosages delivered, scan ranges, contrast injection amounts, radioactive agent injected amounts, isotope activity levels, etc.) may be obtained from the data repositories and used to populate the timelines without the user having to access the data repositories themselves (e.g., in a separate window). In doing so, the radiation timeline as herein disclosed may present a limited set of information to the users in order to increase efficiency of the users' interaction with the available data as users are not forced to sift through separate data feeds, data files, or tabular data tables to identify and then aggregate the needed information.

The systems, methods, and GUIs provided herein may improve the accuracy and timeliness of clinical decision making for care providers in high-demand, high-resource settings (e.g., high-volume medical facility units such as hospital radiology units or oncology units). This may be particularly useful during high-demand situations when there is not adequate time to manually sort through individual tabular data. The time it would take to individually collect data from multiple data repositories, aggregate and analyze the collected data, and visualize the data using standard methods may take inordinate amounts of time due to data volume. By establishing a system that presents relevant data longitudinally, time spent searching for relevant data and making clinical decisions about irradiating imaging examinations for patients may be reduced. Additionally, the timelines as herein noted display alerts in the instance of determined over-irradiation, practice issues, or other alerts, which is not previously seen in radiation dose management systems that use and present tabular data. Further, as data previously presented in multiple areas in tables or otherwise as tabular entries is now aggregated to one timeline, processing power of the computing device may be reduced as less entries need be displayed at once.

As such, the disclosure herein provides a specific way of improving the capability of the healthcare system, by providing one or more GUIs that display data of imaging examinations in a longitudinal manner. The disclosure further provides a specific improvement to the way computers operate by aggregating data from multiple sources in one location, which may obviate the need for users to have to navigate through multiple sources, manually update information, and so forth, thereby increasing the efficiency of the operation of the computer for the user. The radiation timelines and GUIs described herein provide a specific manner of displaying a limited set of information to the user, for example components indicating scan range of individual examination phases (e.g., image series acquisitions, scout acquisitions, etc.) and elements indicating amount of irradiation delivered to those scan ranges. In contrast, prior interface methods display textual tabular data as individual entries, which requires the user to step through multiple tables of exhaustive and in some cases duplicative entries. Thus, the user experience with the computer may be improved and made more efficient.

Furthermore, by displaying a limited set of information via the radiation timelines, operation of the computing device(s) that collect and render the data for display may be improved by reducing the processing demands of the computing device(s), thereby increasing the efficiency of the computing device(s). For example, only certain patient data may be displayed based on a selected imaging study or based on selected or default settings within the GUI (e.g., toggle elements indicating whether or not to display certain data within the timeline), which results in a limited amount of the data that is received being processed for display, which may improve the efficiency of the computing device(s).

The radiation timelines and radiation dose management system herein described may be included as part of a medical information system as described herein and may provide for high speed data processing that allows for display of multiple data types from multiple data repositories, including in some examples multiple types of imaging systems, within a single interface. Thus, via the disclosed radiation dose management system and generated timelines, relational patient history data with regards to administered radiation data for a given imaging study, may be displayed in a manner that is easy to visually parse and act on in a reduced amount of time.

The disclosure also provides support for a computing device comprising a display screen, the computing device being configured to display on the display screen a menu listing one or more data repositories of one or more patients, and additionally being configured to display on the display screen a radiation timeline within a graphical user interface (GUI) accessible from the menu, wherein the radiation timeline displays, for each patient, patient data including one or more examination phases and radiation data thereof of a selected imaging examination, the radiation data and one or more examination phases obtained from the one or more data repositories, wherein each examination phase is plotted along a horizontal axis in chronological order and a component is plotted within the radiation timeline according to a timestamp of a corresponding examination phase and a scan range of the corresponding examination phase, and wherein the GUI is displayed while the one or more data repositories are in an un-launched state. In a first example of the system, the GUI additionally displays a second radiation timeline of examination phases without timestamps. In a second example of the system, optionally including the first example, the GUI additionally displays a radiation dose element that comprises one or more dosage bars indicating an amount of irradiation at various scan ranges, the one or more dosage bars displayed aligned according to the various scan ranges. In a third example of the system, optionally including one or both of the first and second examples, the GUI additionally displays one or more isotope activity elements aligned with the horizontal axis of the radiation timeline, wherein each of the one or more isotope activity elements displays, for each examination phase that corresponds to nuclear medicine, an isotope activity level. In a fourth example of the system, optionally including one or more or each of the first through third examples, the one or more examination phases include one or more of scout acquisitions, image series acquisitions, and injected agent administrations. In a fifth example of the system, optionally including one or more or each of the first through fourth examples, the component is a representation of a scout acquisition when the corresponding examination phase is a scout acquisition step and the representation of the scout acquisition is displayed within the radiation timeline along a vertical axis based on a scan range of the scout acquisition. In a sixth example of the system, optionally including one or more or each of the first through fifth examples, the component is a scan range bar when the corresponding examination phase is an image series acquisition and the scan range bar is displayed within the radiation timeline along a vertical axis based on a scan range of the image series acquisition. In a seventh example of the system, optionally including one or more or each of the first through sixth examples, the one or more data repositories include a plurality of imaging systems, wherein each of the plurality of imaging systems stores in memory radiation data for each examination phase, the radiation data including amount of irradiation delivered for each examination phase.

The disclosure also provides support for a method, comprising: accessing one or more data repositories, retrieving radiation dose data from the one or more data repositories, performing period segmentation to the radiation dose data to segment the radiation dose data into a plurality of examination phases for a selected imaging examination, generating a radiation timeline that displays one or more components for each of the plurality of examination phases, the radiation timeline displaying the one or more components according to time and scan range, and displaying the radiation timeline in a graphical user interface (GUI). In a first example of the method, the one or more components comprise at least one representation of a scout acquisition, wherein the representation of the scout acquisition is displayed within the radiation timeline according to the scan range of the scout acquisition. In a second example of the method, optionally including the first example, the one or more components further comprise one or more sets of scan range components each corresponding to an image series acquisition, wherein each image series acquisition is one of the plurality of examination phases and the one or more sets of scan range components are linked to the at least one representation of the scout acquisition according to respective scan ranges. In a third example of the method, optionally including one or both of the first and second examples, the radiation timeline further comprises isotope activity elements indicating radioactivity level of an injected agent at one or more of the plurality of examination phases. In a fourth example of the method, optionally including one or more or each of the first through third examples, the one or more data repositories comprise one or more of a picture archiving and communication system (PACS), one or more imaging systems, and a radiology information system (RIS), the one or more imaging systems comprising one or more of a computed tomography (CT), mammography, interventional radiology, a positron emission tomography (PET) system, a multi-modality system, a magnetic resonance imaging (MRI) system, an x-ray system, and a radio fluoroscopy (RF) system. In a fifth example of the method, optionally including one or more or each of the first through fourth examples, the plurality of examination phases comprise one or more of scout acquisitions, image series acquisitions, injected agent administrations, and monitoring steps. In a sixth example of the method, optionally including one or more or each of the first through fifth examples, performing period segmentation on the radiation dose data further comprises segmenting data without timestamps from data with timestamps. In a seventh example of the method, optionally including one or more or each of the first through sixth examples, the method further comprises: displaying the data without timestamps in a secondary timeline within the GUI.

The disclosure also provides support for a radiation dose management system, comprising: one or more processors, and memory storing instructions executable by the one or more processors to: retrieve data of an imaging examination of a patient from one or more data repositories, the data including one or more examination phases of the imaging examination and radiation data for each of the one or more examination phases, and output, for display on a display device, a radiation timeline within a graphical user interface (GUI) that includes, the one or more examination phases plotted chronologically and one or more components plotted according to scan range, each corresponding to one of the one or more examination phases. In a first example of the system, the radiation timeline further includes one or more selectable elements that when selected trigger display of a pop-up window displaying additional information relating to the selected element. In a second example of the system, optionally including the first example, the one or more selectable elements comprise one or more of over-irradiation alerts, isotope activity elements, and practice issue alerts. In a third example of the system, optionally including one or both of the first and second examples, the one or more examination phases are plotted as icons along a horizontal axis of the radiation timeline, the icons each being selectable to launch a pop-up window displaying additional information about a corresponding examination phase.

As used herein, an element or step recited in the singular and proceeded with the word "a" or "an" should be understood as not excluding plural of said elements or steps, unless such exclusion is explicitly stated. Furthermore, references to "one embodiment" of the present invention are not intended to be interpreted as excluding the existence of additional embodiments that also incorporate the recited features. Moreover, unless explicitly stated to the contrary, embodiments "comprising," "including," or "having" an element or a plurality of elements having a particular property may include additional such elements not having that property. The terms "including" and "in which" are used as the plain-language equivalents of the respective terms "comprising" and "wherein." Moreover, the terms "first," "second," and "third," etc. are used merely as labels, and are not intended to impose numerical requirements or a particular positional order on their objects.

This written description uses examples to disclose the invention, including the best mode, and also to enable a person of ordinary skill in the relevant art to practice the invention, including making and using any devices or systems and performing any incorporated methods. The patentable scope of the invention is defined by the claims, and may include other examples that occur to those of ordinary skill in the art. Such other examples are intended to be within the scope of the claims if they have structural elements that do not differ from the literal language of the claims, or if they include equivalent structural elements with insubstantial differences from the literal languages of the claims.

## Claims

1. A computing device comprising a display screen, the computing device being configured to display on the display screen a menu listing one or more data repositories of one or more patients, and additionally being configured to display on the display screen a radiation timeline within a graphical user interface (GUI) accessible from the menu, wherein the radiation timeline displays, for each patient, patient data including one or more examination phases and radiation data thereof of a selected imaging examination, the radiation data and one or more examination phases obtained from the one or more data repositories, wherein each examination phase is plotted along a horizontal axis in chronological order and a component is plotted within the radiation timeline according to a timestamp of a corresponding examination phase and a scan range of the corresponding examination phase, and wherein the GUI is displayed while the one or more data repositories are in an un-launched state.

2. The computing device of claim 1, wherein the GUI additionally displays a second radiation timeline of examination phases without timestamps.

3. The computing device of claim 1, wherein the GUI additionally displays a radiation dose element that comprises one or more dosage bars indicating an amount of irradiation at various scan ranges, the one or more dosage bars displayed aligned according to the various scan ranges.

4. The computing device of claim 1, wherein the GUI additionally displays one or more isotope activity elements aligned with the horizontal axis of the radiation timeline, wherein each of the one or more isotope activity elements displays, for each examination phase that corresponds to nuclear medicine, an isotope activity level.

5. The computing device of claim 1, wherein the one or more examination phases include one or more of scout acquisitions, image series acquisitions, and injected agent administrations.

6. The computing device of claim 1, wherein the component is a representation of a scout acquisition when the corresponding examination phase is a scout acquisition step and the representation of the scout acquisition is displayed within the radiation timeline along a vertical axis based on a scan range of the scout acquisition.

7. The computing device of claim **1,** wherein the component is a scan range bar when the corresponding examination phase is an image series acquisition and the scan range bar is displayed within the radiation timeline along a vertical axis based on a scan range of the image series acquisition.

8. The computing device of claim 1, wherein the one or more data repositories include a plurality of imaging systems, wherein each of the plurality of imaging systems stores in memory radiation data for each examination phase, the radiation data including amount of irradiation delivered for each examination phase.

9. A method, comprising:
accessing one or more data repositories;
retrieving radiation dose data from the one or more data repositories;
performing period segmentation to the radiation dose data to segment the radiation dose data into a plurality of examination phases for a selected imaging examination;
generating a radiation timeline that displays one or more components for each of the plurality of examination phases, the radiation timeline displaying the one or more components according to time and scan range; and
displaying the radiation timeline in a graphical user interface (GUI).

10. The method of claim 9, wherein the one or more components comprise at least one representation of a scout acquisition, wherein the representation of the scout acquisition is displayed within the radiation timeline according to the scan range of the scout acquisition.

11. The method of claim 10, wherein the one or more components further comprise one or more sets of scan range components each corresponding to an image series acquisition, wherein each image series acquisition is one of the plurality of examination phases and the one or more sets of scan range components are linked to the at least one representation of the scout acquisition according to respective scan ranges.

12. The method of claim 9, wherein the one or more data repositories comprise one or more of a picture archiving and communication system (PACS), one or more imaging systems, and a radiology information system (RIS), the one or more imaging systems comprising one or more of a computed tomography (CT), mammography, interventional radiology, a positron emission tomography (PET) system, a multi-modality system, a magnetic resonance imaging (MRI) system, an x-ray system, and a radio fluoroscopy (RF) system.

13. The method of claim 9, wherein the plurality of examination phases comprise one or more of scout acquisitions, image series acquisitions, injected agent administrations, and monitoring steps.

14. The method of claim 9, wherein performing period segmentation on the radiation dose data further comprises segmenting data without timestamps from data with timestamps.

15. A radiation dose management system, comprising:
one or more processors; and
memory storing instructions executable by the one or more processors to:
retrieve data of an imaging examination of a patient from one or more data repositories, the data including one or more examination phases of the imaging examination and radiation data for each of the one or more examination phases; and
output, for display on a display device, a radiation timeline within a graphical user interface (GUI) that includes, the one or more examination phases plotted chronologically and one or more components plotted according to scan range, each corresponding to one of the one or more examination phases.
